# EUROPÄISCHE PATENTSCHRIFT

(11) **EP 1 904 240 B1**
(45) Veröffentlichungstag und Bekanntmachung des Hinweises auf die Patenterteilung: **11.11.2009**
(21) Anmeldenummer: 06776157.7
(22) Anmeldetag: 07.07.2006
(51) Int. Cl.: B05B 11/00

(54) **FLUIDAUSTRAGKOPF**
FLUID DISCHARGE HEAD
TETE DE DECHARGE DE FLUIDE

(30) Priorität: 15.07.2005 DE 102005033771
(43) Veröffentlichungstag der Anmeldung: 02.04.2008
(73) Patentinhaber: MeadWestvaco Calmar GmbH, 58675 Hemer (DE)
(72) Erfinder: HARMS, Heiko, 58708 Menden (DE)
(74) Vertreter: Henseler, Daniela
(86) Internationale Anmeldenummer: PCT/EP2006/006663
(87) Internationale Veröffentlichungsnummer: WO 2007/009617

(56) Entgegenhaltungen:
- EP-A1- 0 499 073
- EP-A2- 0 443 192
- EP-A2- 0 815 946
- DE-A1- 19 840 723

## Beschreibung

Die Erfindung betrifft einen Fluidaustragkopf nach dem Oberbegriff des Anspruchs 1.

Fluldaustragköpfe zur Verwendung mit einer Austragvorrichtung sind in vielfältiger Gestaltung aus dem Stand der Technik bekannt.

Aus EP 0 499 073 A1 ist ein gattungsgemäßer Fluidaustragkopf bekannt, der eine Außenstange und eine Innenstange aufweist. Das obere Ende der Außenstange ist mit einer inneren Ausnehmung versehen, die für eine abdichtende Aufnahme eines Kopfes gestaltet ist. Diese Anordnung bildet eine Auslassöffnung. Wenn der Kopf in die Ausnehmung eingreift, ist die Öffnung geschlossen; ist der Kopf von der Ausnehmung getrennt, ist die Öffnung offen. Die Öffnung ist normalerweise geschlossen und wird während des Abwärtshubes geöffnet, wodurch das obere Ende der Außenstange dem Kopf gegenüber nach unten bewegt wird. Die Bewegung der Außenstange nach unten erfolgt entgegen einer Federvorspannung.

Aus EP 0 815 946 A2 ist ein Spender mit einer Keimsperre bekannt. Diese Sperre ist ein druckabhängig öffnendes und schließendes Ventil.

Aus EP 0 443 192 A2 ist ein Austragkopf für Medien bekannt, der einen eine Austragöffnung aufweisenden Austragstutzen umfasst, der mit einer Innenhülse ausgebildet ist. Die Innenhülse nimmt annähernd auf der ganzen Länge den Schaft eines Innenkörpers auf, der eine unmittelbare Verbindung zu einem Gegenstück einer Austragvorrichtung herstellt. Im Wesentlichen wird der gesamte Betätigungsdruck so über den Innenkörper auf das Gegenstück übertragen und Toträume reduziert. Nachteilig ist, dass Keime und sonstige Verunreinigungen durch die Austragöffnung in das System eindringen können.

Aus DE 198 40 723 A1 ist ein Austragkopf für Medien bekannt, der einen Austragstutzen aufweist, dessen Spenderauslass mit einem entgegen der Strömungsrichtung verschließbaren Ventilstöpsel mikrobiologisch dicht schließbar ist. Vor einem Pumpenhub wird das Ventil gegen eine Feder entgegen der Strömungsrichtung geöffnet. Der Schließspalt des Ventils bildet dabei den Medienauslass. Dadurch kann das Medium vor Verkeimung geschützt werden. Nachteilig ist, dass ein derartiger Austragkopf zum Verschluss unmittelbar an der Austragöffnung den Sprühmechanismus beeinträchtigt. Das Ventil behindert zudem die Handhabung.

Aufgabe der Erfindung ist es daher, einen Fluidaustragkopf zu schaffen, der eine gute Handhabung gewährleistet und die Gefahr einer Kontaminierung des Mediums verringert.

Diese Aufgabe wird durch die Merkmale des kennzeichnenden Teils des Anspruchs 1 gelöst.

Hierdurch wird ein Austragkopf geschaffen, bei dem ein Ventil in den Austragstutzen integriert ist. Ein Totvolumen zwischen der Austragöffnung und dem Ventil wird gering gehalten. Die ventilführende Bewegung in axialer Richtung ist als Zwangsbewegung ausgelegt. Somit wird, bevor überhaupt Medium gefördert wird, das Ventil zwangsweise durch die Betätigungskraft des Anwenders geöffnet. Die Innenhülse ist im Bereich ihres stirnseitigen Endes, das die Dichtfläche trägt, bündig in den Austragstutzen eingesetzt, wodurch die Dichtfläche eine stabile Lage erhält.

Das Ventil kann dabei an einer Innenhülse ausgebildet sein, die die auftretenden Betätigungskräfte durch ein Abstützen auf einem Verbindungsglied des Innenkörpers sicher und direkt überträgt, wobei ein Hub zur Öffnung des Ventils über Führungen ausrichtbar ist.

Das Ventil ist klein dimensionierbar, so dass zusätzlich einsatzbare oligodynamische Substanzen keine wesentliche Verteuerung der Herstellung des Austragkopfes zur Folge haben. Sollte eine Keimfreiheit nicht ausreichend gegeben sein, kann also optimal das Ventil durch eingebettete oligodynamische Substanzen in die Lage versetzt werden, Bakterien und Keime wirkungsvoll abzuwehren.

Die Feder zur Federvorspannung kann von einem stirnseitigen Ende des Innenkörpers gebildet werden, so dass sich die Innenhülse stirnseitig direkt an dem Innenkörper abstützen kann. Die Betätigungskräfte wirken dann ebenfalls direkt auf den Innenkörper, der in Abhängigkeit des Federweges die Betätigungskräfte auf das Gegenstück überträgt. Die Betätigungskräfte zum Öffnen des Ventils und zur Übertragung auf das Gegenstück wirken auf den gleichen Innenkörper.

Der Ventilstopfen wird vorzugsweise als Funktionselement für eine Düsenausbildung an der Austrittsöffnung genutzt. Der Ventilstopfen kann dazu als Drallkörper ausgebildet sein. Alternativ kann der Ventilstopfen zur Begrenzung von Dralleinrichtungen im Kopfbereich des Austragstutzens ausgebildet sein. Der Ventilstopfen kann dazu einen Stirnkegel besitzen.

Weitere Ausgestaltungen der Erfindung sind der nachfolgenden Beschreibung und den Unteransprüchen zu entnehmen.

Die Erfindung wird nachfolgend anhand des in den beigefügten Abbildungen dargestellten Ausführungsbeispiels näher erläutert.
Fig. 1 zeigt schematisch einen Axialschnitt eines Fluidaustragkopfes in einer Grundstellung, unbetätigt,
Fig. 2 zeigt schematisch im Axialschnitt den Fluidaustragkopf gemäß Fig. 1 in einer Stellung mit geöffnetem Ventil für einen anschließenden Austragvorgang.

Die Erfindung betrifft einen Fluidaustragkopf zur Verwendung mit einer nicht näher dargestellten Austragvorrichtung, wobei die Austragvorrichtung einen Mediumspeicher für ein Fluid umfasst, in dem das Medium unter Druck gestellt ist oder aus dem das Medium über eine Mediumpumpe, insbesondere einer Schubkolbenpumpe, ausgetragen wird. Die Austragvorrichtung weist ein Gegenstück 9 auf, an dem der Fluidaustragkopf anbringbar ist.

Der Fluidaustragkopf umfasst einen Austragstutzen 1 mit einer Austragöffnung 2, die hier stirnseitig am Austragstutzen 1 angebracht ist. Der Austragstutzen 1 nimmt eine Innenhülse 5 auf, in der ein Innenkörper 4 angeordnet ist, der einen Auslasskanal 7 begrenzt und ein Verbindungsglied 8 zur Verbindung mit dem Gegenstück 9 einer Austragvorrichtung aufweist. Die Innenhülse 5 weist stirnseitig benachbart zur Austragöffnung 2 eine Dichtfläche 10 auf, gegen die ein am Innenkörper 4 befindlicher, den Auslasskanal 7 verschließender Ventilstopfen 3 federvorgespannt ist.

Der Austragstutzen 1 weist für eine manuelle Betätigung unter Aufbringung von Betätigungskräften auf das Gegenstück 9 Fingerauflageflächen 18 auf. Der Austragstutzen 1, der zur Weiterleitung des aus dem Medienbehälter ausgetragenen Fluids dient, schließt mit seinem Auslasskanal 7 an einen Durchlasskanal 19 des Gegenstücks 9. Der Austragstutzen 1 besitzt die Form einer Nasenolive, um als Nasenadapter auf das Gegenstück 9 aufgesetzt werden zu können. Für andere Anwendungszwecke kann der Austragstutzen 1 andere Außenkonturen besitzen.

An dem Endbereich weist der Austragstutzen stirnseitig die Austragöffnung 2 auf, die eine oder mehrere Öffnungen umfassen kann, je nachdem, welches Sprüh-oder Strahlbild erwünscht ist.

Die Innenhülse 5 sitzt fest angeordnet in dem Austragstutzen 1, wobei die Befestigung lösbar über eine Schnappverbindung 20 am Austragstutzen 1 erfolgt.

Die Innenhülse 5 ist im Bereich ihres stirnseitigen Endes, das die Dichtfläche 10 trägt, bündig in den Austragstutzen 1 eingesetzt, wodurch die Dichtfläche 10 eine stabile Lage erhält. Rückseitig der Dichtfläche 10 weist die Innenhülse 5 einen Federsitz 14 für die Federvorspannung des Ventilstopfens 3 auf. Der Federsitz 14 ist ausgebildet an einer einteilig mit der Innenhülse 5 ausgebildeten inneren Buchse 21, die den Auslasskanal 7 gegenüber dem Ventilstopfen 3 bis hin zur Dichtfläche 10 begrenzt. Die Buchse 21 ist vorzugsweise freistehend angeordnet.

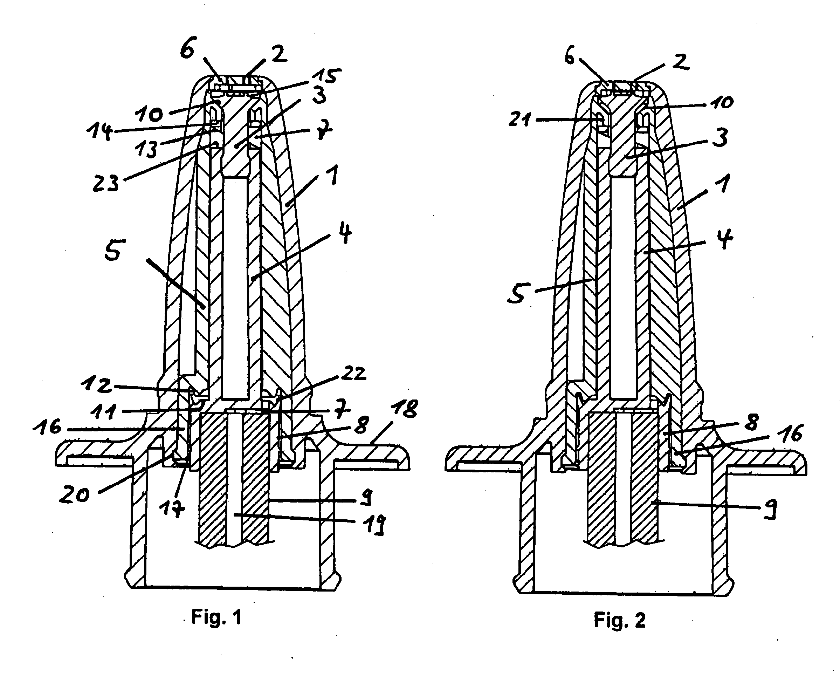

An dem dem Gegenstück 9 zugewandten Ende weist die Innenhülse 5 ein Aufsatzglied 16 auf, über das die Innenhülse 5 am Austragstutzen lösbar befestigt ist. Dieses Aufsatzglied 16 überstülpt das Verbindungsglied 8 des Innenkörpers 4 mit axialem Spiel. Dieses axiale Spiel erlaubt einen axialen Hub des Austragstutzens 1 mit der Innenhülse 5 gegenüber dem Innenkörper 4, um den Ventilstopfen 3 von der Dichtfläche 10 abzuheben, wie dies in Fig. 2 dargestellt ist, bevor das Gegenstück 9 zur Ausgabe von Medium betätigt wird. Das Verbindungsglied 8 bildet einen Anschlag 11 für die Innenhülse 5. Zur Verbesserung des Angriffs der Innenhülse 5 am Verbindungsglied 8 zur Übertragung der Betätigungskräfte trägt diese vorstehende Führungselemente 12, die in formangepasste Vertiefungen 22 am Anschlag 11 des Verbindungsgliedes 8 eingreifen.

Die Innenhülse 5 sitzt gleitverschieblich auf dem Innenkörper 4, der auf dem Gegenstück 9 sitzt. Der Innenkörper 4 begrenzt den Auslasskanal 7 entweder zur Innenhülse 5 oder durch einen inneren Auslasskanal und dient dazu, Toträume im Austragstutzen 1 zu minimieren. Der Innenkörper 4 kann zur Gewichtsreduzierung hohl ausgebildet sein. Für eine mindestens teilweise radiale Mindestbeabstandung des Aufsatzgliedes 16 der Innenhülse 5 weist das Verbindungsglied 8 eine radial vorspringende Führungsstegeinrichtung 17 auf.

Kopfseitig ist an dem Innenkörper 4 der Ventilstopfen 3 angeordnet, hier beispielsweise durch eine Schnappverbindung. Der Ventilstopfen 3 ist demnach fest an den Innenkörper 4 angebunden. Zur Federvorspannung des Ventilstopfens 3 gegenüber der Dichtfläche 10 ist eine Druckfeder 13 zwischen der Rückseite der Dichtfläche 10 und einer Stirnseite 23 des Innenkörpers 4 angeordnet. Vorzugsweise ist die Druckfeder 13 einteilig mit dem Innenkörper 4 ausgebildet, wozu dieser stirnseitig einen Ringscheibenabschnitt tragen kann. Der Federsitz 14 an der Rückseite der Dichtfläche 10 ist an der Buchse 21 ausgebildet. Alternative Formen von Druckfedern sind einsetzbar.

Der Ventilstopfen 3 ist kopfseitig kegelförmig erweitert für die Ausbildung einer trichterförmigen Dichtfläche 10 benachbart zur Austragöffnung 2. Der Ventilstopfen 3 bildet ferner vorzugsweise einen Drallkörper mit stirnseitigen Dralleinrichtungen 15, die bei geöffnetem Ventil, wie in Fig. 2 dargestellt, mit dem stirnseitigen Ende des Austragstutzens 1 einen Düseneinsatz 6 bilden. Alternativ können die Dralleinrichtungen am stirnseitigen Ende des Austragstutzens 1 ausgebildet sein und der Ventilstopfen stirnseitig eine Bodenfläche für den so gebildeten Düseneinsatz bilden.

## Patentansprüche

1. Fluidaustragkopf mit einem eine Austragöffnung (2) aufweisenden Austragstutzen (1), der eine Innenhülse (5) aufnimmt, in der ein Innenkörper (4) angeordnet ist, der einen Auslasskanal (7) begrenzt und ein Verbindungsglied (8) zur Verbindung mit einem Gegenstück (9) einer Austragvorrichtung aufweist, wobei die Innenhülse (5) stirnseitig benachbart zur Austragöffnung (2) eine Dichtfläche (10) aufweist, gegen die ein am Innenkörper (4) befindlicher, den Auslasskanal (7) verschließender Ventilstopfen (3) federvorgespannt ist, **dadurch gekennzeichnet, dass** die Innenhülse (5) im Bereich ihres stirnseitigen Endes, das die Dichtfläche (10) trägt, bündig in den Austragstutzen(1)eingesetzt ist und einteilig mit der Innenhülse (5) eine innere Buchse (21) ausgebildet ist, an der ein Federsitz (14) für die Federvorspannung des Ventilstopfens (3) ausgebildet ist.

2. Fluidaustragkopf nach Anspruch 1, **dadurch gekennzeichnet, dass** zum Abheben des Ventilstopfens (3) von der Dichtfläche (10) die Innenhülse (5) gegenüber dem Innenkörper (4) verschiebbar ist bis zu einem Anschlag (11) am Innenkörper (4) zur Übertragung eines Betätigungsdruckes über den Innenkörper (4) auf das Gegenstück (9).

3. Fluidaustragkopf nach Anspruch 1 oder 2, **dadurch gekennzeichnet, dass** die Innenhülse vorstehende Führungselemente (12) aufweist, die mit dem Anschlag (11) in Eingriff bringbar sind.

4. Fluidaustragkopf nach einem der Ansprüche 1 bis 3, **dadurch gekennzeichnet, dass** zur Federvorspannung eine axial belastbare Druckfeder (13) vorgesehen ist, die sich einerseits an der Innenhülse (5) und andererseits an dem Innenkörper (4) abstützt für eine Zwangsführung der Innenhülse (5) gegenüber dem Ventilstopfen (3).

5. Fluidaustragkopf nach Anspruch 4, **dadurch gekennzeichnet, dass** die Druckfeder (13) ein einstückig mit dem Innenkörper (4) ausgebildeter Ringscheibenabschnitt ist.

6. Fluidaustragkopf nach einem der Ansprüche 1 bis 5, **dadurch gekennzeichnet, dass** die Innenhülse (5) rückseitig der Dichtfläche (10) einen Federsitz (14) für die Federvorspannung des Ventilstopfens (3) aufweist.

7. Fluidaustragkopf nach einem der Ansprüche 1 bis 6, **dadurch gekennzeichnet, dass** der Ventilstopfen (3) in einer von der Dichtfläche (10) abgehobenen Stellung mit der Austragöffnung (2) eine Dralleinrichtung bildet.

8. Fluidaustragkopf nach Anspruch 7, **dadurch gekennzeichnet, dass** der Ventilstopfen (3) einen Drallkörper mit stirnseitigen Dralleinrichtungen (15) bildet, der in Eingriff bringbar ist mit einem Düseneinsatz (6) der Austragöffnung (2).

9. Fluidaustragkopf nach einem der Ansprüche 1 bis 8, **dadurch gekennzeichnet, dass** die Innenhülse (5) ein das Verbindungsglied (8) des Innenkörpers (4) mit axialem Spiel überstülpendes Aufsatzglied (16) aufweist, über das die Innenhülse (5) am Austragstutzen (1) arretierbar ist.

10. Fluidaustragkopf nach Anspruch 9, **dadurch gekennzeichnet, dass** das Verbindungsglied (8) für eine mindestens teilweise radiale Mindest-Beabstandung des Aufsatzgliedes (16) eine Führungsstegeinrichtung (17) aufweist.

## Claims

1. A discharge head for fluids, with a discharge nozzle (1) which has a discharge opening (2) and accommodates an internal sleeve (5) in which is arranged an internal body (4) which delimits an outlet channel (7) and has a connecting element (8) for connection to a counterpart (9) of a discharging apparatus, wherein the internal sleeve (5) has, on the end side adjacent to the discharge opening (2), a sealing surface (10) against which a valve stopper (3) which is located on the internal body (4) and closes the outlet channel (7) is spring prestressed, **characterized in that** the internal sleeve (5) is inserted in the region of its end which bears the sealing surface (10) into the discharge nozzle (1) in a flush manner, and an inner bush (21), on which a spring seat (14) for the spring prestressing of the valve stopper (3) is formed, is formed integrally with the internal sleeve (5).

2. The discharge head for fluids as claimed in claim 1, **characterized in that**, in order to raise the valve stopper (3) from the sealing surface (10), the internal sleeve (5) can be displaced in relation to the internal body (4) as far as a stop (11) on the internal body (4) so as to transmit an actuating pressure to the counterpart (9) via the internal body (4).

3. The discharge head for fluids as claimed in claim 1 or 2, **characterized in that** the internal sleeve has protruding guide elements (12) which can be brought into engagement with the stop (11).

4. The discharge head for fluids as claimed in one of claims 1 to 3, **characterized in that** a compression spring (13) which can be subjected to an axial load is provided for the spring prestressing, the compression spring being supported firstly on the internal sleeve (5) and secondly on the internal body (4) for compulsory guidance of the internal sleeve (5) in relation to the valve stopper (3).

5. The discharge head for fluids as claimed in claim 4, **characterized in that** the compression spring (13) is an annular disk section formed integrally with the internal body (4).

6. The discharge head for fluids as claimed in one of claims 1 to 5, **characterized in that**, on the rear side of the sealing surface (10), the internal sleeve (5) has a spring seat (14) for the spring prestressing of the valve stopper (3).

7. The discharge head for fluids as claimed in one of claims 1 to 6, **characterized in that** the valve stopper (3), in a position in which it is raised from the valve surface (10), forms a swirling device together with the discharge opening (2).

8. The discharge head for fluids as claimed in claim 7, **characterized in that** the valve stopper (3) forms a swirling body with swirling devices (15) on the end side, which swirling body can be brought into engagement with a nozzle insert (6) of the discharge opening (2).

9. The discharge head for fluids as claimed in one of claims 1 to 8, **characterized in that** the internal sleeve (5) has an attachment element (16) which can be pulled with axial play over the connecting element (8) of the internal body (4) and via which the internal sleeve (5) can be locked to the discharge nozzle (1).

10. The discharge head for fluids as claimed in claim 9, **characterized in that** the connecting element (8) has a guide web device (17) for an at least partial radial minimum spacing of the attachment element (16).

## Revendications

1. Tête de décharge de fluide, avec un manchon de décharge (1) présentant une ouverture de décharge (2), qui incorpore une douille interne (5), dans laquelle est agencé un corps interne (4), qui délimite un canal de sortie (7) et présente un élément de liaison (8) pour relier à une pièce correspondante (9) d'un dispositif d'écoulement, la douille interne (5) présentant une surface d'étanchéité (10) au voisinage externe de l'ouverture de décharge (2), contre laquelle une valve-bouchon (3), se trouvant au niveau du corps interne (4) et fermant le canal de sortie (7), est précontrainte élastiquement, **caractérisée en ce que**
la douille interne (5) est enfoncée nettement dans le manchon de décharge (1) au niveau de son extrémité frontale, qui porte la surface d'étanchéité (10), et qu'une douille intérieure (21) est formée solidairement à la douille interne (5), contre laquelle est agencé un logement élastique (14) pour la précontrainte élastique de la valve-bouchon (3).

2. Tête de décharge de fluide selon la revendication 1, **caractérisée en ce que** la douille interne (5) est mobile par rapport au corps interne (4) jusqu'à une butée (11) au niveau du corps interne (4) pour transmettre une pression d'actionnement du corps interne (4) sur la partie correspondante (9) afin de soulever la valve-bouchon (3) de la surface d'étanchéité (10).

3. Tête de décharge de fluide selon la revendication 1 ou 2, **caractérisée en ce que** la douille interne présente des éléments de guidage (12) protubérants, qui peuvent être accouplés à la butée (11).

4. Tête de décharge de fluide selon l'une des revendications 1 à 3, **caractérisée en ce qu'**un ressort compressible axialement (13), qui s'appuie d'une part sur la douille interne (5) et d'autre part sur le corps interne (4) pour un guidage forcé de la douille interne (5) vers la valve-bouchon (3), est prévu pour la précontrainte élastique.

5. Tête de décharge de fluide selon la revendication 4, **caractérisée en ce que** le ressort (13) forme une section en forme d'anneau monobloc avec le corps interne (4).

6. Tête de décharge de fluide selon l'une des revendications 1 à 5, **caractérisée en ce que** la douille interne (5) présente un logement élastique (14) pour la précontrainte élastique de la valve-bouchon (3) au revers de la surface d'étanchéité (10).

7. Tête de décharge de fluide selon l'une des revendications 1 à 6, **caractérisée en ce que** la valve-bouchon (3) constitue, dans une position dégagée de la surface d'étanchéité (10), un dispositif de torsion par rapport à l'ouverture de décharge (2).

8. Tête de décharge de fluide selon la revendication 7, **caractérisée en ce que** la valve-bouchon (3) constitue un corps de torsion avec un dispositif de torsion (15) à façades, qui peut être accouplé avec un insert de buse (6) de l'ouverture de décharge (2).

9. Tête de décharge de fluide selon l'une des revendications 1 à 8, **caractérisée en ce que** la douille interne (5) présente un élément chapeautant (16) se mettant sur l'élément de liaison (8) du corps interne (4) avec un jeu axial, au dessus duquel la douille interne (5) peut être arrêtée au niveau du manchon de décharge (1).

10. Tête de décharge de fluide selon la revendication 9, **caractérisée en ce que** l'élément de liaison (8) présente un dispositif à barrette de guidage (17) pour fournir au moins partiellement un écart radial minimal de l'élément chapeautant (16).
